# EUROPÄISCHE PATENTSCHRIFT

(11) **EP 1 696 810 B1**
(45) Veröffentlichungstag und Bekanntmachung des Hinweises auf die Patenterteilung: **23.01.2019**
(21) Anmeldenummer: 03776750.6
(22) Anmeldetag: 22.12.2003
(51) Int. Cl.: A61B 17/80, A61B 17/86

(54) **KNOCHENPLATTE**
BONE PLATE
PLAQUE POUR OSTEOSYNTHESE

(43) Veröffentlichungstag der Anmeldung: 06.09.2006
(73) Patentinhaber: Synthes GmbH, 4436 Oberdorf (CH)
(72) Erfinder: BEUTTER, Florian, CH-4500 Solothurn (CH); PAULI, Werner, CH-4950 Huttwil (CH)
(74) Vertreter: Klunker IP Patentanwälte PartG mbB
(86) Internationale Anmeldenummer: PCT/CH2003/000838
(87) Internationale Veröffentlichungsnummer: WO 2005/060846

(56) Entgegenhaltungen:
- DE-A- 10 003 968
- DE-A1- 4 201 043
- FR-A- 2 836 369
- US-B1- 6 206 883
- US-B1- 6 315 852
- US-B1- 6 605 090

## Beschreibung

Die Erfindung bezieht sich auf eine Knochenplatte gemäss dem Oberbegriff des Patentanspruchs 1.
Aus der US 5,868,746 SARVER ist eine gattungsgemässe Knochenplatte bekannt. Dieses Dokument offenbart eine X-förmige Knochenplatte, welche an ihren vier Extremitäten vier Bohrungen aufweist. Nachteilig bei dieser bekannten X-Platte ist der Umstand, dass die vier Bohrungen keine Mittel aufweisen, um die darin einzuführenden Schrauben in rigider, winkelstabiler Weise mit der Platte zu verbinden.
Aus der deutschen Offenlegungsschrift DE-A 40 38 082 OJIMA ist eine Doppel-T-förmige Platte bekannt, welche vier Plattenlöcher mit einem Gewinde zeigt, welche entsprechende Gewindekopfschrauben aufnehmen können. Nachteilig bei dieser Platte ist der Umstand, dass sie nicht X-förmig ausgebildet ist. Sie weist deshalb bei Biegung oder Torsion entlang ihrer Längsachse eine schlechte Spannungsverteilung auf kann deshalb hauptsächlich nur in einer Richtung, nämlich auf Druck, belastet werden.

US 6 315 852 B1 und DE 42 01 043 A1 offenbaren X-förmige Knochenplatten.

Hier will die Erfindung Abhilfe schaffen. Der Erfindung liegt die Aufgabe zugrunde, eine Knochenplatte zu schaffen, welche in der Lage ist, zwei Knochen oder Knochenfragmente in jeder Hinsicht stabil zu fixieren. Eine weitere Aufgabe besteht darin eine optimale Kombination von Resistenz gegenüber Biegung und Rotation sowie gegen Druckbelastung bei minimaler Invasivität zu erzielen.
Die Erfindung löst die gestellte Aufgabe mit einer Knochenplatte, welche die Merkmale des Anspruchs 1 aufweist.
Die durch die Erfindung erreichten Vorteile sind im wesentlichen darin zu sehen, dass
A) eine günstige Spannungsverteilung erreicht wird im Vergleich zu H- oder Doppel-T-Platten,
B) eine erhöhte und vor allem rigide Stabilität durch die winkelstabile Verankerung der Schrauben in der Platte ermöglicht wird; und
C) die Möglichkeit besteht je 2 Schrauben pro Knochen, bzw. Knochenfragment oder Osteotomiepartner einzubringen und dadurch Rotationsstabilität zu erhalten.
Bei einer besonderen Ausführungsform weist mindestens eine der Plattenbohrungen eine Zentralachse auf, welche nicht parallel zur Normalen auf dem Mittelteil (2) steht. Dadurch wir bei einem Anbiegen der Knochenplatte über eine der beiden Mittelachsen verhindert, dass die in die Plattenbohrungen eingeführten Schrauben miteinander kollidieren. Insbesondere die mindestens eine der Plattenbohrung mit Mitteln kann eine Zentralachse aufweisen, welche nicht parallel zur Normalen auf dem Mittelteil steht. Eine der winkelstabilen Schrauben kann damit so gesetzt werden, dass sie über die Osteotomie, bzw. Fraktur geht, was der Verbindung der beiden Knochenfragmente eine erhöhte Stabilität verleiht. Ein Aufbiegen der Knochenplatte bei ungünstigen Kräfteverhältnissen kann so verhindert werden.
Bei einer weiteren Ausführungsform beträgt Anzahl N der Plattenbohrungen mit Mitteln, welche eine Zentralachse aufweisen, die nicht parallel zur Normalen auf dem Mittelteil steht, zwei, drei oder vier.
Mindestens ein Teil der Plattenbohrungen kann sich gegen die knochenseitige Unterseite hin konisch verjüngen, so dass dadurch die Mittel, die zur rigiden, winkelstabilen Verankerung von darin einzuführenden Knochenschrauben geeignet sind, realisiert sind. Diese Mittel bestehen aber auch aus einem Innengewinde. Bei einer weiteren Ausführungsform sind vier Plattenbohrungen an den Ecken eines Rechtecks angeordnet. Dadurch kann eine erhöhte Rotationsstabilität realisiert werden durch das Einbringen von je zwei Schrauben pro Knochenfragment.
Die Plattenbohrungen können auch Zentralachsen aufweisen, welche nicht parallel zueinander verlaufen. Dies hat den Vorteil, dass Schrauben verwendet werden können, die abgewinkelt in die Plattenbohrungen eingebracht sind, um die Osteotomie, Knochenfraktur oder das Gelenk zu kreuzen, so dass die Stabilität auch von der Knochenschraube selbst kommt und nicht nur von der Knochenplatte in Verbindung mit der Knochenschraube.

Die Zentralachsen der Plattenbohrungen können auch unterhalb der knochenseitigen Unterseite der Knochenplatte gegeneinander konvergieren. Dadurch kann vermieden werden, dass die in die Knochenplatte eingeführten Schrauben beim Verbiegen der Platte miteinander kollidieren.

Die Zentralachsen er Plattenbohrungen können sich auch in einem Punkt schneiden. Dadurch kann die Knochenplatte in jeder Richtung verwendet werden, ohne auf eine spezielle Abwinkelung achten zu müssen. Die Normale im Zentrum des Mittelteils der Knochenplatte kann ebenfalls durch diesen Punkt verlaufen.

Bei einer weiteren Ausführungsform liegt die Knochenplatte in einer Ebene. Sie kann im weiteren zwei orthogonal aufeinander stehende Symmetrieebenen aufweisen.

Zusammen mit der Knochenplatte können mindestens zwei Knochenschrauben verwendet werden, welche mit einem Kopf und einem Schaft versehen sind, wobei der Kopf mindestens einer der Knochenschrauben Mittel aufweist, die eine rigide, winkelstabilen Verankerung in den Plattenbohrungen gestattet. Vorzugsweise kann der Kopf der mindestens einen Knochenschraube konisch ausgebildet sein. Der Kopf der Knochenschraube kann auch ein Aussengewinde tragen. Der Kopf der mindestens einen Knochenschraube kann auch aus einem härteren Material bestehen als die Knochenplatte im Bereich der Plattenbohrung, wobei der Kopf ein Aussengewinde besitzt, welches dann beim Eindrehen in die Plattenbohrung ein entsprechendes Gewinde einschneidet.
Alle drei Ausführungen (Konus, Aussengewinde, härteres Material) des Schraubenkopfes stellen Mittel dar, die eine rigide, winkelstabilen Verankerung in den Plattenbohrungen gestattet.

Vorteilhafterweise weist das durch die vier Plattenbohrungen aufgespannten Viereck eine Höhe von 10 bis 40 mm und ein Breite von 10 bis 40 mm auf.

Mindestens ein Teil der von den vier Armen des "X" gebildeten Winkel können abgerundet sein, damit sich eine bessere Spannungsverteilung durch Abflachung der Spannungsspitzen ergibt.

Bei einer weiteren Ausführungsform weist die Knochenplatte im Bereich der Plattenlöcher gegenüber den Bereichen zwischen den Plattenlöchern eine grössere Dicke auf. Dadurch wird die Knochenplatte noch weniger invasiv. Im Bereich um die Plattenlöcher herum kann hingegen wegen des konischen Schraubenkopfs eine gewisse Minimalhöhe nicht unterschritten werden. Ein weiterer Vorteil besteht darin, dass bei einem Anbiegen der Knochenplatte die Plattenlöcher nicht oder nur minimal deformiert werden.

Bei einer weiteren Ausführungsform sind die vier Arme des "X" der Knochenplatte gegenüber dem Mittelteil abbiegbar, so dass sie der Knochenkontur angepasst werden können.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer X-förmigen Knochenplatte mit vier konischen Plattenlöchern;
Fig. 2 eine Aufsicht auf die Knochenplatte nach Fig. 1;
Fig. 3 eine perspektivische Ansicht einer Variante der Knochenplatte nach Fig. 1 mit darin eingesetzten Knochenschrauben; und
Fig. 4 eine perspektivische Ansicht einer Knochenschraube nach Fig. 3.

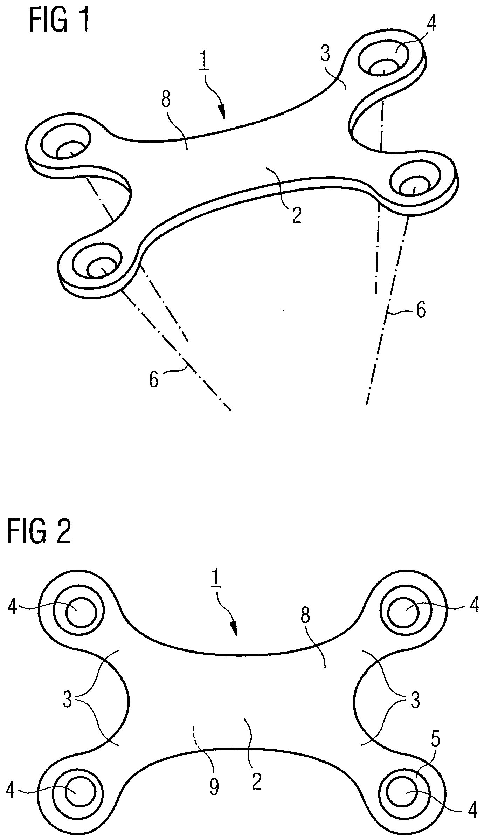

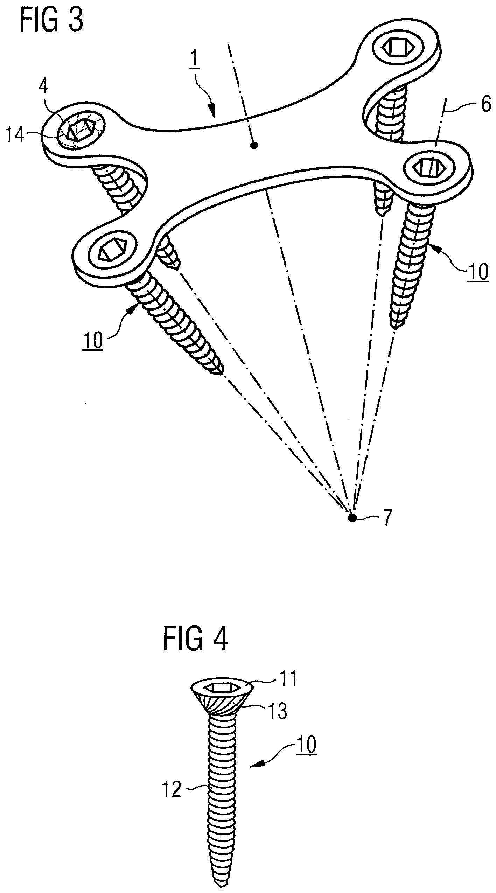

Die in den Fig. 1 und 2 dargestellte Knochenplatte 1 umfasst eine Oberseite 8, eine knochenseitige Unterseite 9, ein Mittelteil 2 und einem daran anschliessenden peripheren Bereich 3, in welchem vier Plattenbohrungen 4 untergebracht sind. Die vier Plattenbohrungen 4 weisen Mittel 5 auf, die zur rigiden, winkelstabilen Verankerung von darin einzuführenden Knochenschrauben 10 geeignet sind. Diese Mittel 5 bestehen darin, dass sich die Plattenbohrungen 4 gegen die knochenseitige Unterseite 9 hin konisch verjüngen. Knochenschrauben 10 mit einem entsprechend konisch ausgebildeten Kopf 11 können dann winkelstabil und in rigider Weise mit der Knochenplatte 1 verbunden werden.
Die Mittel 5 können - wie in Fig. 3 dargestellt - zusätzlich ein Innengewinde 14 umfassen.
Die Knochenplatte 1 besitzt die Form eines "X", wobei der periphere Bereich 3 die vier Arme des "X" umfasst. Die vier Plattenbohrungen 4 sind am Ende der vier Arme des "X" an den Ecken eines gedachten Rechtecks angeordnet. Die von den vier Armen des "X" gebildeten Winkel sind abgerundet.

Die vier Plattenbohrungen 4 weisen Zentralachsen 6 auf, welche unterhalb der knochenseitigen Unterseite 9 der Knochenplatte 1 gegeneinander konvergieren und sich im wesentlichen in einem Punkt 7 schneiden. Die Normale im Zentrum des Mittelteils 2 der Knochenplatte 1 verläuft durch den Punkt 7 (Fig. 3). Die Knochenplatte 1 weist somit zwei orthogonal aufeinander stehende Symmetrieebenen auf.

Wie in Fig. 3 dargestellt kann die Knochenplatte 1 vier Knochenschrauben 10 (Fig. 4) aufnehmen, welche mit einem Kopf 11 und einem Schaft 12 versehen sind, wobei der Kopf 11 Mittel 13 in Form eines konischen Aussengewindes aufweist, die eine rigide, winkelstabilen Verankerung in den Plattenbohrungen 4 gestatten.

Die Knochenplatte 1 liegt in einer Ebene und kann vom Benutzer nach Belieben angebogen werden. Sie kann aber auch bereits vorgebogen dem Benutzer zur Verfügung gestellt werden.

## Patentansprüche

1. Knochenplatte (1), welche die Form eines "X" aufweist, mit einer Oberseite (8), einer knochenseitigen Unterseite (9), einem Mittelteil (2) und einem daran anschliessenden peripheren Bereich (3), welcher die vier Arme des "X" umfasst und in welchem mindestens vier Plattenbohrungen (4) untergebracht sind, wobei die vier Plattenbohrungen (4) am Ende der vier Arme des "X" angeordnet sind, und
die Enden der vier Arme des "X" je eine Breite A aufweisen und mit dem Mittelteil (2) mittels Verbindungsbereichen mit je einer Breite B verbunden sind, wobei die Breite B der Verbindungsbereiche kleiner als die Breite A der Enden der vier Arme des "X" ist, wobei mindestens eine der Plattenbohrungen (4) Mittel (5) aufweist, die zur rigiden, winkelstabilen Verankerung von darin einzuführenden Knochenschrauben (10) geeignet sind, wobei die Mittel (5) aus einem Innengewinde (14) bestehen.

2. Knochenplatte (1) nach Anspruch 1, **dadurch gekennzeichnet, dass** mindestens eine der Plattenbohrungen (4) eine Zentralachse (6) aufweist, welche nicht parallel zur Normalen auf dem Mittelteil (2) steht.

3. Knochenplatte (1) nach Anspruch 2, **dadurch gekennzeichnet, dass** die mindestens eine Plattenbohrung (4) mit Mitteln (5) eine Zentralachse (6) aufweist, welche nicht parallel zur Normalen auf dem Mittelteil (2) steht.

4. Knochenplatte (1) nach Anspruch 3, **dadurch gekennzeichnet, dass** die Anzahl N der Plattenbohrungen (4) mit Mitteln (5), welche eine Zentralachse (6) aufweisen, die nicht parallel zur Normalen auf dem Mittelteil (2) steht, zwei, drei oder vier beträgt.

5. Knochenplatte (1) nach einem der Ansprüche 1 bis 4, **dadurch gekennzeichnet, dass** sich mindestens ein Teil der Plattenbohrungen (4) gegen die knochenseitige Unterseite (9) hin konisch verjüngen und dadurch die Mittel (5) realisiert sind.

6. Knochenplatte (1) nach einem der Ansprüche 1 bis 5, **dadurch gekennzeichnet, dass** die vier Plattenbohrungen (4) an den Ecken eines Rechtecks angeordnet sind.

7. Knochenplatte (1) nach einem der Ansprüche 1 bis 6, **dadurch gekennzeichnet, dass** die Plattenbohrungen (4) Zentralachsen (6) aufweisen, welche nicht parallel zueinander verlaufen.

8. Knochenplatte (1) nach Anspruch 7, **dadurch gekennzeichnet, dass** die Zentralachsen (6) unterhalb der knochenseitigen Unterseite (9) der Knochenplatte (1) gegeneinander konvergieren.

9. Knochenplatte (1) nach Anspruch 8, **dadurch gekennzeichnet, dass** sich die Zentralachsen (6) im wesentlichen in einem Punkt (7) schneiden.

10. Knochenplatte (1) nach Anspruch 9, **dadurch gekennzeichnet, dass** die Normale im Zentrum des Mittelteils (2) durch den Punkt (7) verläuft.

11. Knochenplatte (1) nach einem der Ansprüche 1 bis 10, **dadurch gekennzeichnet, dass** sie in einer Ebene liegt.

12. Knochenplatte (1) nach einem der Ansprüche 1 bis 11, **dadurch gekennzeichnet** sie zwei orthogonal aufeinander stehende Symmetrieebenen aufweist.

13. Knochenplatte (1) nach einem der Ansprüche 1 bis 12, **dadurch gekennzeichnet, dass** das durch die vier Plattenbohrungen (4) aufgespannten Viereck eine Höhe von 10 bis 40 mm aufweist.

14. Knochenplatte (1) nach einem der Ansprüche 1 bis 13, **dadurch gekennzeichnet, dass** das durch die vier Plattenbohrungen (4) aufgespannten Viereck eine Breite von 10 bis 40 mm aufweist.

15. Knochenplatte (1) nach einem der Ansprüche 1 bis 14, **dadurch gekennzeichnet, dass** mindestens ein Teil der von den vier Armen des "X" gebildeten Winkel abgerundet sind.

16. Knochenplatte (1) nach einem der Ansprüche 1 bis 15, **dadurch gekennzeichnet, dass** sie im Bereich der Plattenlöcher (4) gegenüber den Bereichen zwischen den Plattenlöchem eine grössere Dicke aufweist.

17. Knochenplatte (1) nach einem der Ansprüche 1 bis 16, **dadurch gekennzeichnet**, die vier Arme des "X" gegenüber dem Mittelteil (2) abbiegbar sind.

18. Vorrichtung umfassend eine Knochenplatte (1) nach einem der Ansprüche 1 bis 17, und mindestens zwei Knochenschrauben (10), welche mit einem Kopf (11) und einem Schaft (12) versehen sind, wobei der Kopf (11) mindestens einer der Knochenschrauben (10) Mittel (13) aufweist, die eine rigide, winkelstabilen Verankerung in den Plattenbohrungen (4) gestattet.

19. Vorrichtung nach Anspruch 18, **dadurch gekennzeichnet, dass** der Kopf (11) der mindestens einen Knochenschraube (10) ein Aussengewinde (13) trägt und als Mittel (13) wirkt.

## Claims

1. Bone plate (1), which has the shape of an "X", having an upper side (8), a bone-side lower side (9), a central part (2) and a peripheral region (3) adjoining thereto which comprises the four arms of the "X" and in which at least four plate bores (4) are accommodated, wherein the four plate bores (4) are arranged at the end of the four arms of the "X" and the ends of the four arms of the "X" each have a width A and are connected with the central part (2) by means of connecting regions of a respective width B, wherein the width B of the connecting regions is smaller than the width A of the ends of the four arms of the "X", wherein at least one of the plate bores (4) has means (5) that are suitable for the rigid, angularly stable anchoring of bone screws (10) to be introduced therein, wherein the means (5) are formed of an internal thread (14).

2. The bone plate (1) according to claim 1, **characterized in that** at least one of the plate bores (4) has a central axis (6) that is not parallel to the normal on the central part (2).

3. The bone plate (1) according to claim 2, **characterized in that** the at least one plate bore (4) with means (5) has a central axis (6) that is not parallel to the normal on the central part (2).

4. The bone plate (1) according to claim 3, **characterized in that** the number N of plate bores (4) with means (5) having a central axis (6) that is not parallel to the normal on the central part (2) amounts to two, three or four.

5. The bone plate (1) according to any of claims 1 to 4, **characterized in that** at least a portion of the plate bores (4) tapers conically towards the bone-side lower side (9) and thereby the means (5) are realized.

6. The bone plate (1) according to any of claims 1 to 5, **characterized in that** the four plate bores (4) are arranged at the corners of a rectangle.

7. The bone plate (1) according to any of claims 1 to 6, **characterized in that** the plate bores (4) have central axes (6) that do not extend parallel to one another.

8. The bone plate (1) according to claim 7, **characterized in that** the central axes (6) below the bone-side lower side (9) of the bone plate (1) converge towards each other.

9. The bone plate (1) according to claim 8, **characterized in that** the central axes (6) intersect substantially at a point (7).

10. The bone plate (1) according to claim 9, **characterized in that** the normal extends in the center of the central part (2) through the point (7).

11. The bone plate (1) according to any of claims 1 to 10, **characterized in that** it lies in a plane.

12. The bone plate (1) according to any of claims 1 to 11, **characterized in that** it has two planes of symmetry disposed orthogonally to one another.

13. The bone plate (1) according to any of claims 1 to 12, **characterized in that** the quadrangle spanned by the four plate bores (4) has a height of 10 to 40 mm.

14. The bone plate (1) according to any of claims 1 to 13, **characterized in that** the quadrangle spanned by the four plate bores (4) has a width of 10 to 40 mm.

15. The bone plate (1) according to any of claims 1 to 14, **characterized in that** at least a part of the angles formed by the four arms of the "X" are rounded.

16. The bone plate (1) according to any of claims 1 to 15, **characterized in that** it has a greater thickness in the region of the plate holes (4) than with respect to the regions between the plate holes.

17. The bone plate (1) according to any of claims 1 to 16, **characterized in that** the four arms of the "X" are bendable with respect to the central part (2).

18. An apparatus comprising a bone plate (1) according to any of claims 1 to 17, and at least two bone screws (10) that are supplied with a head (11) and a shaft (12), wherein the head (11) of at least one of the bone screws (10) has means (13) that allow a rigid, angularly stable anchoring in the plate bores (4).

19. The apparatus according to claim 18, **characterized in that** the head (11) of the at least one bone screw (10) bears an external thread (13) and acts as the means (13).

## Revendications

1. Plaque d'ostéosynthèse (1) en forme de X ayant une face supérieure (8), une face inférieure (9) côté os, une partie médiane (2) et une zone périphérique (3) unie à cette dernière, laquelle comprend les quatre branches du X et dans laquelle sont ménagés au moins quatre alésages de plaque (4), cependant que les quatre alésages de plaque (4) sont agencés à l'extrémité des quatre branches du X et que les extrémités des quatre branches du X présentent une largeur A et sont jointes à la partie médiane (2) au moyen de zones de jonction ayant chacune une largeur B, cependant que la largeur B des zones de jonction est inférieure à la largeur A des extrémités des quatre branches du X,
cependant qu'au moins un des alésages de plaque (4) comporte des moyens (5) appropriés à l'ancrage rigide et angulairement stable de vis à os (10) à y introduire, cependant que les moyens (5) consistent en un filetage intérieur (14).

2. Plaque d'ostéosynthèse (1) selon la revendication 1, **caractérisée en ce qu'**au moins un des alésages de plaque (4) présente un axe central (6) non parallèle à la normale sur la partie médiane (2).

3. Plaque d'ostéosynthèse (1) selon la revendication 2, **caractérisée en ce que** le moins un alésage de plaque (4) ayant des moyens (5) présente un axe central (6) non parallèle à la normale sur la partie médiane (2).

4. Plaque d'ostéosynthèse (1) selon la revendication 3, **caractérisée en ce que** le nombre N des alésages de plaque (4) ayant des moyens (5) qui présentent un axe central (6) non parallèle à la normale sur la partie médiane (2) s'élève à deux, trois ou quatre.

5. Plaque d'ostéosynthèse (1) selon une des revendications de 1 à 4, **caractérisée en ce qu'**au moins une partie des alésages de plaque (4) s'effilent coniquement vers la face inférieure (9) côté os et que les moyens (5) sont ainsi réalisés.

6. Plaque d'ostéosynthèse (1) selon une des revendications de 1 à 5, **caractérisée en ce que** les quatre alésages de plaque (4) sont agencés aux angles d'un rectangle.

7. Plaque d'ostéosynthèse (1) selon une des revendications de 1 à 6, **caractérisée en ce que** les alésages de plaque (4) présentent des axes centraux (6) non parallèles entre eux.

8. Plaque d'ostéosynthèse (1) selon la revendication 7, **caractérisée en ce que** les axes centraux (6) convergent entre eux en-dessous de la face inférieure (9) côté os de la plaque d'ostéosynthèse (1).

9. Plaque d'ostéosynthèse (1) selon la revendication 8, **caractérisée en ce que** les axes centraux (6) se croisent essentiellement en un point (7).

10. Plaque d'ostéosynthèse (1) selon la revendication 9, **caractérisée en ce que** la normale, au centre de la partie médiane (2), passe par le point (7).

11. Plaque d'ostéosynthèse (1) selon une des revendications de 1 à 10, **caractérisée en ce qu'**elle se trouve dans un plan.

12. Plaque d'ostéosynthèse (1) selon une des revendications de 1 à 11, **caractérisée en ce qu'**elle présente deux plans de symétrie orthogonaux entre eux.

13. Plaque d'ostéosynthèse (1) selon une des revendications de 1 à 12, **caractérisée en ce que** le quadrilatère défini par les quatre alésages de plaque (4) présente une hauteur de 10 à 40 mm.

14. Plaque d'ostéosynthèse (1) selon une des revendications de 1 à 13, **caractérisée en ce que** le quadrilatère défini par les quatre alésages de plaque (4) présente une largeur de 10 à 40 mm.

15. Plaque d'ostéosynthèse (1) selon une des revendications de 1 à 14, **caractérisée en ce qu'**au moins une partie des angles formés par les quatre branches du X sont arrondis.

16. Plaque d'ostéosynthèse (1) selon une des revendications de 1 à 15, **caractérisée en ce que**, dans la zone des orifices de plaque (4), elle présente une épaisseur plus grande par rapport aux zones situées entre les orifices de plaque.

17. Plaque d'ostéosynthèse (1) selon une des revendications de 1 à 16, **caractérisée en ce que** les quatre branches du X peuvent être recourbées par rapport à la partie médiane (2).

18. Dispositif comprenant une plaque d'ostéosynthèse (1) selon une des revendications de 1 à 17, et au moins deux vis à os (10) pourvues d'une tête (11) et d'une tige (12), cependant que la tête (11) d'au moins une des vis à os (10) comporte un moyen (13) qui permet un ancrage rigide et angulairement stable dans les alésages de plaque (4).

19. Dispositif selon la revendication 18, **caractérisée en ce que** la tête (11) de la au moins une vis à os (10) porte un filetage extérieur (13) et agit comme moyen (13).
